(19) Europäisches Patentamt
European Patent Office
Office européen des brevets

(11) EP 0 415 783 B1

(12) **EUROPEAN PATENT SPECIFICATION**

(45) Date of publication and mention of the grant of the patent:
**17.07.1996 Bulletin 1996/29**

(51) Int Cl.6: **D01D 10/02**, D01D 5/16, A61L 17/00

(21) Application number: **90309553.7**

(22) Date of filing: **31.08.1990**

# (54) Thermal treatment of thermoplasticc filaments and filaments

Wärmebehandlung von thermoplastischen Filamenten und Filamente

Traitement thermique de filaments thermoplastiques et filaments

(84) Designated Contracting States:
**BE DE ES FR GB IT LU NL SE**

(30) Priority: **01.09.1989 US 402092**

(43) Date of publication of application:
**06.03.1991 Bulletin 1991/10**

(73) Proprietor: **ETHICON INC.**
**Somerville New Jersey 08876 (US)**

(72) Inventor: **Broyer, Ephraim**
**Murray Hill, N.J. 07974 (US)**

(74) Representative: **Fisher, Adrian John et al**
**CARPMAELS & RANSFORD**
**43 Bloomsbury Square**
**London WC1A 2RA (GB)**

(56) References cited:
**GB-A- 1 588 031**
**US-A- 4 052 988**
**US-A- 4 629 654**
**US-A- 4 911 165**

- **PATENT ABSTRACTS OF JAPAN vol. 10, no. 355 (C-388)(2411) November 29, 1986 & JP-A-61152810 (CHISSO CORP.) July 11, 1986**

EP 0 415 783 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

The present invention relates to the production of thermoplastic filaments by extrusion and drawing, and more particularly, to a process for improving the physical properties of such thermoplastic monofilaments by thermal treatment. This invention also relates to monofilaments produced by the disclosed process, which monofilaments are particularly useful as surgical sutures.

Background of the Invention

Monofilaments are prepared from many different thermoplastic polymers for a variety of industrial and professional applications. The physical properties of such monofilaments, such as tensile strength, elongation and modulus, depend on the particular polymer composition and on the method of manufacture. It is known, for example, that melt spun nylon and polyethylene terephthalate polymers produce monofilaments of higher tenacity than, for example, the polyolefins, and that the tenacity of such monofilaments is increased while elongation is decreased by drawing the filaments under conditions to increase molecular orientation.

US-A-4629654 discloses vinylidene fluoride resin monofilaments, particularly for fishing lines, fishing nets and ropes. The abrasion resistance of the filaments is said to be improved by heat treatment to relax the orientation of the surface part of the resin.

The use of thermoplastic monofilaments as surgical sutures is well established. Monofilament sutures formed of isotactic polypropylene are described in U.S. 3,630,205, and monofilament sutures of poly-dioxanone are described in U.S. 4,052,988.

Copolymers of p-dioxanone and glycolide useful in preparing monofilament sutures are described in U.S. 4,635,497, while sutures comprising poly [tetramethylene terephthalate-co-(alkenyl or alkyl) succinate] are described in U.S. 4,388,426. Other polymers suggested for use as synthetic absorbable sutures are disclosed in the literature and are well-known to those skilled in the art.

It is generally desirable for surgical sutures to posses high tenacity (greater than 50,000 psi), low to moderate elongation (from 20 - 60%), and low modulus (less than 500,000 psi). Low modulus values signify a high degree of filament flexibility and limpness as opposed to a stiff, wiry material, a particularly desirable and even essential feature of a surgical suture. U.S. 3,630,205 describes a process whereby the flexibility of polypropylene sutures may be improved with little sacrifice in tensile strength by stretching the monofilament about 6.6x under controlled conditions and then relaxing to 76 - 91% of the stretched length.

U.S. 4,246,904 describes a surgical suture prepared from a segmented polyether-ester block copolymer which is reported to have excellent strength and flexibility compared to prior art monofilament sutures. The sutures of poly(p-dioxane) described in U.S. 4,052,988 are also characterized as possessing good tensile and knot strength and a high level of flexibility and softness.

The desirability of suture flexibility and softness as indicated by low modulus values is well recognized by the surgical profession and is a physical property constantly sought after in connection with the development of new surgical suture products. Some suture materials, such polylactide, polyglycolide, and copolymers of lactide and glycolide are generally considered to be too stiff and wiry to be used as monofilaments in all but the smallest suture sizes, and have found commercial acceptance in the larger sizes only as braided sutures, which are more flexible by virtue of their physical construction.

It is accordingly an object of the present invention to provide a process whereby monofilaments having improved softness and flexibility may be prepared. It is a further object of this invention to provide a process for reducing the modulus of existing monofilament materials with little or no loss of tensile strength. It is yet a further object of this invention ot provide improved surgical monofilament sutures of poly(p-dioxanone) and other suitable polymeric materials characterized by reduced values of Young's modulus. A further object of this invention is to provide a method whereby the surface characteristics of thermoplastic monofilaments may be modifies to enhance the properties of such monofilaments, particularly surface smoothness and integrity. These and other objects will be apparent from the ensuing description and claims.

Summary of the Invention

In the basic embodiment of the present invention, drawn and oriented thermoplastic monofilaments are subjected to a heat treatment to reduce modulus and otherwise improve physical properties by passing the filament through a radiant heater maintained above the melting temperature of the monofilament. Operating conditions are controlled so that the monofilament is subjected to a sufficient time/temperature exposure to modify the near-surface crystalline structure of the monofilament.

The monofilament is maintained under tension and preferably drawn slightly during the heat treatment. Draw ratios of 10 - 20 percent or higher are possible with most materials. Treatment temperature may be 5° to 100°C or more above the melting temperature of the monofilament, with exposure time adjusted to obtain the desired effect on crystalline structure without penetrating too deeply within the monofilament. Following the heat treatment, the monofilament is relaxed and annealed to further increase crystallinity and decrease the degree of amorphous orientation.

In a further embodiment of the present invention, melt extruded, liquid quenched monofilaments are drawn through a radiant heater maintained at a temperature above the melting temperature of the monofilaments. Draw speed, draw ratio, heater temperature and dwell time are regulated to obtain the maximum stable draw ratio for the particular monofilament material. This draw ratio will generally by 3x to 6x and 10 to 30 percent more than the maximum stable draw ratio obtainable in the absence of the radient heater. The use of the radiant heater to increase the overall stretch imparted to the filament during the initial drawing and orientation step further improves the ultimate physical properties obtainable by the method of this invention. The filaments thus produced are further processed according to the basic embodiment of the invention.

Monofilaments, drawn and heat treated in accordance with the present invention, are further processed through relaxation and annealing following conventional procedures. The resulting filaments are found to have significantly greater elongation, significantly lower modulus and substantially equivalent tensile strength as compared to monofilaments processed without the heat treating step of the present invention.

## Description of Drawing

Fig. 1 is a schematic representation of a filament extrusion process according to the present invention.

Fig. 2 is a schematic representation of a filament redraw process according to the present invention.

Fig. 3 is a cross-section photomicrograph of a size 4/0 monofilament suture of poly(p-dioxanone) prepared according to the method of the present invention.

Fig. 4 is a cross-section photomicrograph of a size 4/0 monofilament suture of poly(p-dioxanone) prepared according to the prior art.

Fig. 5 is a cross-section photomicrograph of a size 6/0 monofilament suture of poly(p-dioxanone) prepared according to the method of the present invention.

Fig. 6 is a cross-section photomicrograph of a size 6/0 monofilament suture of poly(p-dioxanone) prepared according to the prior art.

## Detailed Description of the Invention

In one embodiment of the present invention, there is provided a process for drawing and relaxing prepared filaments in order to modify and improve the properties of such monofilaments. The filaments subjected to such process have already been prepared by conventional methods of melt extruding, quenching, drawing, and relaxing continuous thermoplastic monofilaments. This embodiment of the invention is referred to herein as the "redraw process".

In a second embodiment of the present invention, there is provided an improved process for the initial preparation of the filaments which are to be further processed by the redraw process. This embodiment of the invention, hereinafter referred to as the "extrusion process", in combination with the redraw process, constitutes a preferred embodiment of total invention and results in monofilament products having optimum properties.

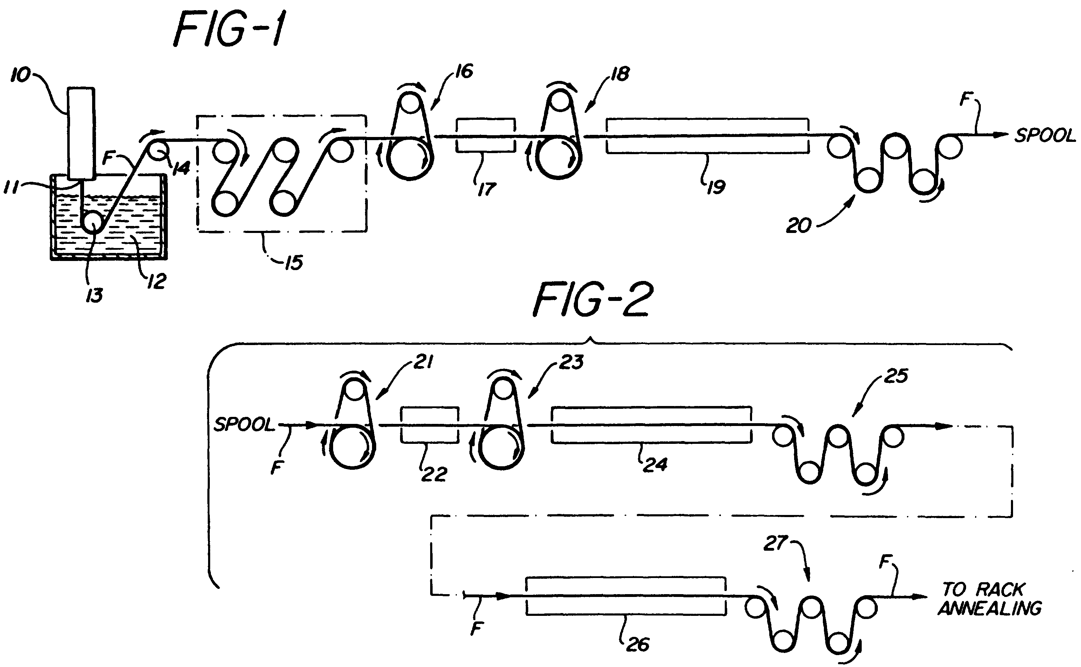

The extrusion process of the present invention is first described as follows with reference to Fig. 1. The selected thermoplastic polymer is melt extruded using conventional melt spinning apparatus indicated generally as 10. The polymer passes from the spinnette 11 having one or more jet orifices sized to provide the correct final diameter of the desired monofilament into aqueous quench bath 12 where the molten polymer stream hardens into filament F. The filament passes around submerged roll 13 and exits the quench bath over roll 14, thereafter passing into air cabinet 15 where the filament is dried and conditioned before entering the drawing portion of the process. The roll speed in cabinet 15 may be adjusted to take up the filament at a rate faster than the filament is being extruded from the orifice in which case the rolls impart a "jet-stretch" to the filament. The amount of jet stretch, together with orifice size, is adjusted to produce a monofilament of the desired diameter.

The filaments pass from air cabinet 15 to draw rolls 16 which are generally maintained at the same peripheral speed as the rolls in the cabinet so the filaments are maintained under tension but not significantly drawn at this point. The filaments leaving draw rolls 16 pass through tube furnace 17 onto draw rolls 18 which operate at a higher peripheral speed than draw rolls 16 in order to impart a high degree of stretch to the filaments, i.e., 3x - 6x (200 - 500%). The tube furnace, which is optional at this point in the present invention, is operated at a temperature of from 5 to 100° above the melting temperature of the filaments, and most generally, from 20 to 75° above the melting point. Because the furnace is relatively short and the time of filament exposure is short, only the surface of the filament is affected by the heat. The use of the tube furnace at this point in the process allows a higher degree of stretch to be imparted to the filament, but for some polymer systems, the furance may be omitted and the filament given a cold, warm air or hot water stretch. In any case, the drawing at this stage imparts molecular orientation to the filaments which are essentially amorphous or unoriented spherolitic structures going onto rolls 16.

After drawing, the filaments pass through hot air orientation oven 19, maintained at a temperature between the Tg and the melting point of the polymer, where they are given a small additional stretch of e.g. 10 - 25% by draw rolls 20. This completes the initial filament preparation stage of the process and the filaments are spooled and stored for further processing in the redraw process of the present invention.

The redraw process is next described with reference to Fig. 2 as follows. Filaments F prepared and spooled in the extrusion process or obtained from other suitable source are fed to draw roll 21, through tube furnace 22, and onto draw rolls 23. The filament is drawn usually less than 100% and most generally from 10 to 25% between rolls 21 and 23. The tube furnace, which is an essential component of the redraw process, is operated at a temperature at least 5°C above the melting point of the filaments, and preferably at least 15° or more above the melting point. In general, the maximum temeprature which is consistent with good operation is preferred and the optimum temperature for any given process will be dependent on filament size and composition, operating speed, and draw ratios. Such optimum temperatures are readily determined by experimental procedures for any given system.

The drawn and heat treated filament proceeds from rolls 23 through hot air conditioning oven 24 and on to draw rolls 25 which operate at a peripheral speed slightly above that of rolls 23, e.g. 2-10%, in order to maintain the filaments under tension. The filaments next proceed from rolls 25 through hot air relaxing oven 26 and onto rolls 27. Rolls 27 operate at a peripheral speed which is 10 to 30% slower than that of rolls 25 in order to permit the filaments to shrink a controlled amount while passing through oven 26. The temperature in oven 24 and oven 26 are maintained above the Tg but below the melting temperature of the filament, usually about 5 to 20°C below the melting point.

Filaments leaving rolls 27 are collected and optionally subjected to further processing if desired, such as annealing to increase crystallinity. The particular further processing steps, if any, will depend to a large extent on the composition of the filament and the physical properties which are desired.

The tube furnace, which is an essential component of the redraw process, and an optionally preferred component of the extrusion process, is preferably a high temperature furnace which heats by radiant energy as well as convection. A suitable furnace for single or small groups of monofilaments is tube furnace Model No. 55035 available from the Lindberg Co., 304 Mart Street, Watertown, Wisconsin. This furnace is designed to reach temperatures of up to 900°C, and is heated by electrical elements backed by a refractory material. The furnace has a 2.5 cm (1-inch) diameter tubular opening through which the filaments pass. It is important when using this furnace that the filaments be centered in the opening to avoid uneven heating. For larger scale multifilament production, a furnace with infrared heaters and parallel heating plate geometry would be preferred to a tubular furnace.

The method of the present invention is illustrated by the following example describing the preparation of a monofilament suture of poly(p-dioxanone).

Poly(p-dioxanone) sutures are known from U.S. 4,052,988, incorporated herein by reference. The preparation of p-dioxanone monomer and polymerization of that monomer to obtain polymers having intrinsic viscosities in excess of 0.80 are described in this reference. Further, the extrusion of the polymer according to Example VI of this reference resulted in monofilaments having a diameter of 0.28 mm (11 mils) (corresponding to a 3/0 suture) a tensile strength of $3.8 \times 10^5$ kPa (55,600 psi), a dry knot strength of $3.4 \times 10^5$ kPa (48,800 psi), and a Youngs modulus of $1.15 \times 10^6$ kPa (167,000 psi). Example VII describes a 0.23 mm (9 mil) monofilament having a tensile strength of $4.9 \times 10^5$ kPa (70,600 psi), dry knot strength of $3.5 \times 10^5$ kPa (50,300 psi), and an elongation of 46.3 percent.

A commercial monofilament suture comprising a polymer of p-dioxanone and manufactured and sold by Ethicon, Inc., Somerville, New Jersey, under the trademark "PDS® Suture", has been determined to have average properties as shown in Table I below.

Poly(p-dioxanone) sutures size 2/0 and 5/0 were produced from the same polymer as aforedescribed commercial sutures by the method of the present invention. The physical properties of these sutures are also presented in Table I below. As illustrated by these data, the sutures produced according to the present invention have similar tensile and knot strength, greater elongation, and significantly lower modulus than the commercial sutures.

TABLE I

| Suture Properties | | | | |
|---|---|---|---|---|
| | This Invention | | Commercial Product | |
| Suture Size | 5/0 | 2/0 | 5/0 | 2/0 |
| Diameter mm (mil) | 0.17 (6.8) | 0.4 (13.5) | 0.17 (6.5) | 0.35 (13.7) |
| Tensile kg (lb) | 1.5 (3.4) | 5.7 (12.6) | 1.3 (2.9) | 5.0 (11.1) |
| Intr. ten. kPa x $10^{-5}$ (psi) | 6.4 (92,770) | 6.1 (88,775) | 6.0 (87,550) | 5.2 (75,010) |
| Elong. (%) | 58.0 | 60.0 | 38.1 | 34.1 |
| Knot kg (lb) | 1.0 (2.3) | 3.3 (7.3) | 1.0 (2.1) | 3.2 (7.0) |
| Intr. knot kPa x $^{-5}$ (psi) | 4.4 (63,130) | 3.5 (51,280) | 4.3 (62,000) | 3.3 (47,140) |
| Modulus kPa x $10^{-6}$ (psi) | approx. 1.4 (200,000) | | approx. 2.3 (330,000) | |

The reduced modulus and increased elongation of the sutures of the present invention impart improved handling characteristics and reduced tissue drag which are readily perceived and preferred by surgeons using these materials. In particular, the reduced modulus results in greater flexibility and less springiness than that characteristic of the commercial product. The poly(p-dioxanone) sutures of the present invention were found to have essentially the same in vivo breaking strength retention and absorption characteristics as the commercial product.

The sutures of the present invention reported in Table I were prepared by the process as generally described below. This process is described specifically for the manufacture of poly(p-dioxanone) sutures wherein the starting polymer has an average molecular weight of from about 65,000 to 100,000, an inherent viscosity of from about 1.60 to 2.20, and a melting temperature of from about 95 to 105°C. Process conditions may vary for other polymer compositions including other poly(p-dioxanone) polymers and the following example is accordingly presented for purposes of illustration only as being representative for one specific polymer system and is not otherwise limiting of the present invention. Many variations in the process equipment and operating parameters which nevertheless incorporate the essential features of the present invention will be apparent to those skilled in the art and are intended to be included within the scope of the present invention.

EXAMPLE (size 2/0)

Poly(p-dioxanone) polymer having an average MW of about 78,000, an inherent viscosity of 1.65 - 1.80 (melting point approx. 95°C) was melt extruded through a 1.5mm (60 mil) orifice and quenched in a water bath at 24°C. The extruded filament was conditioned to about 80°C in a warm air cabinet and fed to a draw station where it was drawn 5x between two set of godets while passing through a tube furnace at 125°C. The first godet speed was 4.0m/min (13 FPM), the second godet speed was 20m/min (65 FPM), and the furnace was 30.5 cm (12 inches) long. Filament dwell time in the furnace was estimated to be about 1 second. The drawn filament was drawn an additional 20% while passing through a hot air oven maintained at about 80°C. The filament was collected and conditioned under vacuum at room temperature for 16 hours. The monofilament was next drawn 15% while passing through a 30.5 cm (12 inch) tube furnace maintained at about 115 - 120°C. Dwell time in the furnace was estimated to be approximately 1.5 seconds. The drawn filament was next passed through a hot air oven maintained at about 90°C with a 4% stretch to maintain tension, followed by a second oven at 90°C wherein the filament was allowed to shrink 15%. The resulting filament was wound on a rack for annealing at a temperature of from about 70 to 90°C, and, in the present example, at 85°C for 6 hours while maintained under tension to prevent further shrinkage. The resulting monofilament had a diameter of 0.36 mm (14 mils) corresponding to a size 2/0 surgical suture.

The above procedure was repeated to prepare sutures ranging in size from 7/0 (10µm; 0.4 mils) to 2 (0.58mm; 23 mils). Individual suture sizes were obtained by adjusting extrusion rates and/or orifice size to produce an extruded monofilament which, after passing through the remainder of the process which resulted in a 6x overall stretch, would produce the desired suture diameter. While minor adjustments in process speeds and temperatures were made to accommodate the different filament sizes and to optimize process conditions for each size, the basic process was the same as that described above for the size 2/0 suture.

Representative physical properties for sutures prepared in accordance with the present invention as described above are presented in Table II together with comparable data for commercial sutures of the same polymeric compositions. As noted above, the commercial sutures were prepared by a conventional process which did not include heat treatment at a temperature above the melting point of the polymer.

TABLE II

Physical Properties of Sutures of Poly(p-dioxanone)

| Suture Size | This Invention: Straight Tensile kg(lbs) | This Invention: Knot Strength kg(lbs) | This Invention: Elong. (%) | This Invention: Strain Load | Commercial Product: Straight Tensile kg(lbs) | Commercial Product: Knot Strength kg(lbs) | Commercial Product: Elong (%) | Commercial Product: Strain Load |
|---|---|---|---|---|---|---|---|---|
| 2 | 12.7(28.1) | 7.4(16.4) | 59.4 | 2.7 | 13.6(29.9) | 7.3(16.1) | 37.1 | 3.9 |
| 1 | 10.3(22.8) | 5.9(12.9) | 54.7 | 2.2 | 11.1(24.5) | 6.0(13.2) | 27.1 | 3.1 |
| 0 | 8.1(17.8) | 4.7(10.3) | 58.3 | 1.6 | 8.0(17.6) | 4.8(10.5) | 36.9 | 2.4 |
| 2/0 | 5.0(11.0) | 3.3(7.2) | 52.0 | 1.0 | 4.9(10.9) | 3.1(6.8) | 32.3 | 1.8 |
| 3/0 | 3.5(7.7) | 2.5(5.5) | 51.6 | 0.8 | 3.7(8.1) | 2.4(5.2) | 34.5 | 1.2 |
| 4/0 | 2.1(4.6) | 1.5(3.3) | 53.7 | 0.4 | 2.2(4.8) | 1.5(3.2) | 32.1 | 0.7 |
| 5/0 | 1.3(2.9) | 1.0(2.2) | 44.6 | 0.3 | 1.4(3.0) | 1.0(2.1) | 33.3 | 0.4 |
| 6/0 | 0.6(1.3) | 0.5(1.0) | 50.6 | 0.1 | 0.6(1.3) | 0.4(0.9) | 29.1 | 0.2 |
| 7/0 | 0.4(0.8) | 0.3(0.7) | 52.6 | 0.1 | 0.3(0.6) | 0.3(0.6) | 26.1 | 0.1 |

As illustrated by the data in Table II, which are generally representative of these products, sutures prepared according to the present invention are substantially equivalent to the commercial sutures in straight tensile and knot strength, but have significantly higher elongation. In addition, the sutures of the present invention possess a higher degree of elasticity as indicated by the Strain Load which is a measure of the force required to reach the 3% strain level in a stress-strain test. The lower the strain load value, the more stretchy the suture is perceived to be.

Sutures of poly(p-dioxanone) were also evaluated for tensile strength, modulus, and Gurley Stiffness with the representative results presented in Table III below.

TABLE III

Physical Properties of Sutures of Poly(p-dioxanone)

| Nominal Suture Size | This Invention: Diameter mm (mils) | Gurley Stiffness | Ten. kPa x $10^{-5}$ (psi x $10^{-3}$) | Mod. kPa x $10^{-5}$ (psi x $10^{-3}$) | Ten./Mod. | Commercial Product: Diameter mm (mils) | Gurley Stiffness | Ten. kPa x $10^{-5}$ (psi x $10^{-3}$) | Mod. kPa x $10^{-5}$ (psi x $10^{-3}$) | Ten./Mod. |
|---|---|---|---|---|---|---|---|---|---|---|
| 2 | 0.59(23.3) | 240 | 4.4(63.7) | 1.45(210) | 0.30 | 0.60(23.8) | 364 | 5.0(72.5) | 2.3(336) | 0.22 |
| 1 | 0.53(20.8) | 149 | -- | -- | -- | 0.50(19.6) | 169 | 5.2(75.7) | 2.2(318) | 0.24 |
| 0 | 0.47(18.4) | 85 | 4.8(69.1) | 1.38(200) | 0.35 | 0.43(17.0) | 118 | 5.0(71.8) | 2.2(323) | 0.22 |
| 2/0 | 0.36(14.1) | 30 | 4.8(70.2) | 1.37(198) | 0.35 | 0.35(13.6) | 54 | 5.1(73.4) | 2.3(337) | 0.21 |
| 3/0 | 0.30(12.0) | 16 | 5.1(74.2) | 1.40(204) | 0.36 | 0.29(11.6) | 23 | 5.2(75.3) | 2.3(333) | 0.23 |
| 4/0 | 0.21(8.4) | 5.6 | -- | -- | -- | 0.22(8.7) | 7.0 | 5.7(82.3) | 2.4(354) | 0.23 |
| 5/0 | 0.17(6.6) | 1.8 | 5.7(82.6) | 1.37(199) | 0.43 | 0.18(6.9) | 2.6 | 5.3(77.2) | 2.3(334) | 0.23 |
| 6/0 | 0.11(4.4) | 0.2 | -- | -- | -- | 0.11(4.4) | 0.2 | 6.2(89.6) | 2.2(322) | 0.28 |
| 7/0 | 0.01(0.4) | 0.04 | -- | -- | -- | 0.01(0.4) | 0.04 | -- | 2.4(351) | -- |

As shown by the above data, the sutures prepared according to the present invention have significantly lower modulus than the commercial product, approximately 1.4 x $10^6$ kPa (200,000 psi) vs. 2.3 x $10^6$ kPa (330,000 psi). This lower modulus is also reflected in the Gurley Stiffness values which are significantly lower for the sutures of the present invention compared to the commercial product. In this particular study, the tensile strength of the test sutures was slightly lower than that of the commercial product. Further process refinement and optimization of operating conditions however, would be expected to produce further improvement of the physical properties of sutures prepared according to the present invention, including increased tensile strength. Particularly preferred sutures of poly(p-dioxanone) prepared according to the present invention are specifically characterized by the following physical properties:

Elongation - greater than 40%
Young's Modulus - less than 1.7 x $10^6$ kPa (250,000 psi)
Tenacity/Modulus ratio - greater than 0.30

Test methods used for determining the physical properties of monofilament suture materials were as follows:

Tensile strength, knot strength and percent elongation at break were determined by A.S.T.M. method D-2256-66T at a constant rate of extension using an INSTRON table Model 4200 universal testing instrument manufactured by the Instron Corporation of Canton, Massachusetts. With the instrument sample clamps set 12.7cm (5 inches) apart, 12.7 cm (5-inch) lengths of suture were elongated at a rate of 30.5 cm (12 inches) per minute until fracture. The suture knot strength was determined under the same test conditions.

The INSTRON instrument was set for the correct suture diameter, and Young's Modulus was calculated in psi from the initial stress-strain data generated during the straight tensile strength test. Young's Modulus is the ratio of applied stress to strain in the elastic region of the suture and measures the elastic component of a suture's resistance to stress. This value is related to the flexibility of a suture.

Gurley stiffness was measured using a motor-operated Gurley Stiffness Tester (Model 4171-D) manufactured by Teledyne - Gurley of Troy, New York. In the test, 10 parallel monofilaments are mounted on a fixture so that the filaments extend precisely 1.9 cm (3/4 inch) beyond the edge of the fixture. The instrument is adjusted to provide 1.3 cm (1/2 inch) clearance between the edge of the fixture and the pendulum so that the pendulum acts against 0.6 cm (1/4 inch) of the monofilaments. The instrument is operated and filament stiffness computed according to the manufacturer's instructions.

Good suture flexibility as indicated by low Gurley Stiffness values is important to the surgeon since soft, highly flexible sutures are easier to handle, use and tie. Flexible and slightly elastic sutures are also desirable since they conform to the wound and permit some latitude in the tension applied to the suture by the surgeon.

In addition to their desirable physical properties, monofilaments produced according to the present invention are characterized by a unique morphology which includes a central core having a highly ordered crystalline structure and an annular portion between the central core and outer surface which has a less ordered crystalline structure. This differentiation in crystalline structure is believed to be the result of exposing the filament to high temperature in excess of the melting point of the polymer for brief periods during the manufacturing process as described above. While applicant does not wish to be bound by theory, it is postulated that the surface of the filament may actually melt as the filament passes through the heater section, and the penetration of heat into the body of the filament results in the difference in crystalline structure noted above. The thickness of the annular portion which may be from about 0.1 to 0.5x the radius of the filament is a measure the degree of heat penetration which is a function of the time and temperature of exposure.

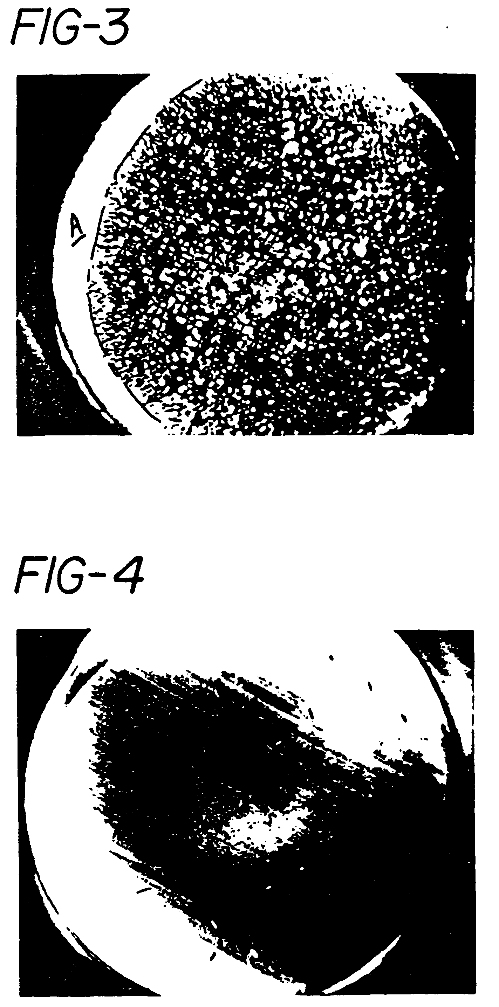

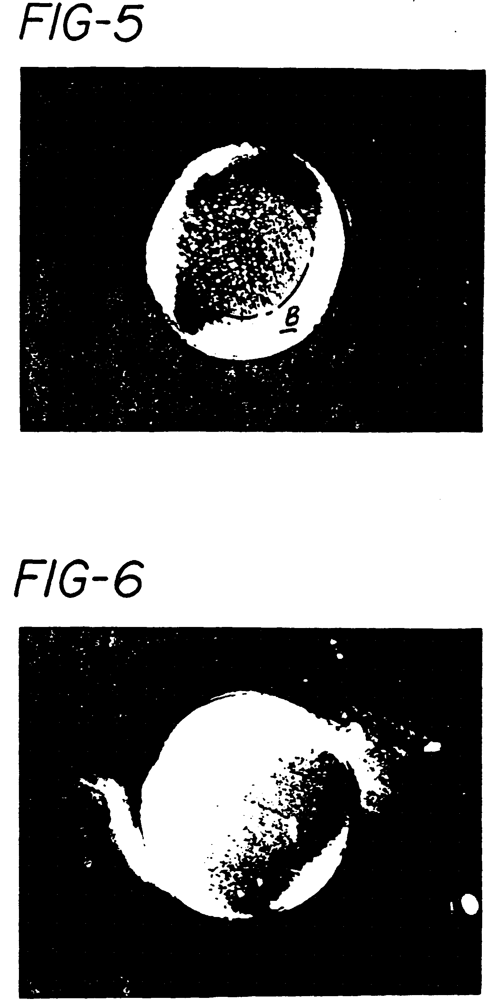

The morphological differences between the poly(p-dioxanone) monofilaments produced according to the present invention and the commercial sutures of the same polymeric composition are readily apparent in Fig. 3 and Fig. 4, which are 430x photomicrographs of cross-sections of size 4/0 filaments illuminated with polarized light. The commercial product illustrated in Fig. 4 is seen to have a relatively uniform crystalline structure throughout its cross-section. The monofilament of the present invention illustrated in Fig. 3 is seen to have a clearly defined central core surrounded by annular area A which extends inward from the surface of the filament a distance equal to about 0.2x the radius of the filament. The crystalline structure of the central core appears to be highly ordered and characterized by large, possibly spherolitic crystals. The annular area surrounding the core appears to have appreciably smaller crystals indicative of a less ordered structure. This observed difference in crystalline structure is theoretically consistent with the effect of the different processing conditions used in the manufacture of the two products. The same effect is seen in Fig. 5 and Fig. 6 which are respectively 430x photomicrographs of size 6/0 sutures of the present invention and commercial product. In Fig. 5, annular area B extends inward from the surface of the filament a distance equal to about 0.4 x the radius of the filament.

The differences in crystalline structure between the poly(p-dioxanone) suture of the present invention and the commercial product are readily determined by x-ray diffraction patterns and scattering analysis as illustrated by the representative values shown in Table IV.

TABLE IV

| Crystalline Properties of Poly(p-dioxanone) Sutures | | | | | | | | |
|---|---|---|---|---|---|---|---|---|
| | This Invention | | | | Commercial Product | | | |
| Suture Size | $X_c$ | L | $\ell_s$ | $F_h$ | $X_c$ | L | $\ell_s$ | $F_h$ |
| 2/0 | 0.52 | 78 | 95 | 206 | 0.47 | 64 | 96 | 229 |
| 4/0 | 0.55 | 72 | 97 | 208 | 0.52 | 61 | 97 | 245 |
| 6/0 | 0.56 | 76 | 97 | 180 | 0.51 | 71 | 99 | 213 |

where:

$X_c$ is the relative crystallinity calculated as

$$X_c = \Sigma I_c / \Sigma I_t$$

where $\Sigma I_c$ is the integrated area under the crystalline peaks, and $\Sigma I_t$ is the integrated area under the observed diffraction curve.

L is an estimate of the breadth in Å of individual crystallites using the Debye-Scherrer equation

$$L = \lambda / \beta \cos \theta$$

where $\theta$ is half the diffraction angle, $\lambda$ is the wave length of radiation in Å, and $\beta$ is the peak width in Å at half-height.

$\ell_s$ is a measure of the long range crystalline structure determined by small angle radiation scatter as the average distance in Å from the center of one crystallite to the center of the next measured in the chain direction along the fiber axis.

$F_n$ is a measure of the long range crystallite perfection in the chain direction along the fiber axis as indicated by crystalline peak width in Å at half-height using the Debye-Scherrer equation as defined above.

The method of the present invention has been illustrated with specific reference to monofilament sutures of poly (p-dioxanone) having unique morphology and improved physical properties, and such sutures are part of the present invention. The method of the present invention however, has wide application for use with other thermoplastic polymeric compositions and for the preparation of monofilamentary materials for industrial as well as surgical applications. Such polymeric compositions which are contemplated for use in the method of the present invention include polyethylene, polypropylene, polyvinylidene fluoride, poly(glycolide, cotrimethyl carbonate), homopolymers and copolymers of lactide and glycolide and copolymers of p-dioxanone with lactide, glycolide and ε-caprolactone.

Many variations in the details of the process of the present invention will be apparent to those skilled in the art. The process may, for example, incorporate further drawing, relaxing and annealing stages in addition to those disclosed herein or may omit or modify one or more of the nonessential processing steps described herein. In addition, the essential feature of the present invention which involves heat treating a monofilament comprising a thermoplastic polymer at a temperature in excess of the melting point of that polymer, is subject to variation according to the composition of the polymer, the size of the monofilament, and the time/temperature relationship of the heat treating step. For example, while the preceeding description included temperatures as high as 75°C above the melting point of the polymer, and exposure times as brief as 0.1 seconds, it may be desirable for higher production speeds or for other purposes to reduce exposure times to 0.01 seconds or less while increasing the temperature up to several hundred degrees above the melting point of the polymer as necessary to obtain the desired effect on the morphology of the monofilament and its physical properties. It is accordingly understood that this invention is not limited to the specifically described embodiments, but is as defined in the claims appended hereto.

## Claims

1. A method for producing a monofilament structure of a thermoplastic polymeric composition comprising the steps of melt extruding and quenching the polymer to form a continuous filament, drawing the filament to achieve molecular orientation, and exposing the filament to a temperature in excess of the melting temperature of the polymeric composition during or subsequent to drawing said filament, wherein that said filament is relaxed or annealed subsequent to said drawing and exposure, and in that said exposure is for a time and at a temperature effective to obtain a filament having an outer surface, a central core, and an annular portion between said central core and said outer surface, said central core having a crystalline structure which is differentiated from that of said annular portion by having a more highly ordered crystalline structure than said annular portion, or larger crystals than said annular portion, and said differentiation between said central core and said annular portion being readily visible by optical microscopy at 430x magnification under polarized light.

**2.** The method of claim 1 wherein said exposure occurs during the first drawing of the extruded and quenched filament.

**3.** The method of claim 1 wherein said exposure occurs after the first drawing of said extruded and quenched filament, and said filament is maintained under tension during said exposure.

**4.** The method of claim 1 wherein said filament is exposed a first time during a first drawing of said extruded and quenched filament, and is exposed a second time during a second drawing of said drawn filament.

**5.** The method of claim 1 wherein said first drawing is in excess of 200 percent and said second drawing is less than 100 percent.

**6.** The method of claim 1, wherein the annular portion has a radial thickness of from about 0.1 to 0.5x the radius of the filament.

**7.** The method of claim 1 wherein said temperature of exposure is from about 5 to 100°C above the melting temperature of said polymer.

**8.** The method of claim 7 wherein the time of said exposure is from about 0.1 to 5 seconds.

**9.** The method of claim 1 wherein said thermoplastic polymer is selected from the group consisting of polyethylene, polypropylene, polyvinylidene fluoride, poly(glycolide, cotrimethyl carbonate), homopolymers and copolymers of lactide and glycolide and copolymers of p-dioxanone with lactide, glycolide and ε-caprolactone.

**10.** The method of claim 1 wherein said polymer is poly(p-dioxanone).

**11.** The method of claim 10 wherein said extruded and quenched monofilament is drawn 4 to 6x while being exposed to a temperature of from about 105°C to 175°C for a period of from about 0.1 to 5 seconds.

**12.** The method of claim 11 wherein said filament is drawn 5x while being exposed to a temperature of about 120 to 140°C.

**13.** The method of claim 11 wherein said drawn and exposed filament is redrawn about 5 to 30% while being exposed a second time to a temperature of from about 105 to 175°C for a period of from about 0.1 to 5 seconds.

**14.** The method of claim 13 wherein said redrawn and exposed filament is relaxed by shrinking 10 to 25% of its redrawn length at a temperature below its melting point.

**15.** The method of claim 14 wherein said filament is annealed after relaxation at a temperature of from about 70 to 90°C while being restrained against further shrinkage.

**16.** A drawn and oriented monofilament structure comprising a thermoplastic polymer and having a structure defined by an outer surface, a central core, and an annular portion between said central core and said outer surface, said annular portion having a radial dimension of from about 0.1 to 0.5x the radius of the monofilament, said central core having a crystalline structure which is differentiated from that of said annular portion by having a more highly ordered crystalline structure than said annular portion, or larger crystals than the annular portion, and said differentiation between said central core and said annular portion being readily visible by optical microscopy at 430x magnification under polarized light.

**17.** The monofilament of claim 16, wherein said polymer is selected from the group consisting of polyethylene, polypropylene, polyvinylidene fluoride, poly(glycolide, cotrimethyl carbonate), homopolymers and copolymers of lactide and glycolide and copolymers of p-dioxanone with lactide, glycolide and ε-caprolactone.

**18.** The monofilament of claim 16 wherein said polymer is poly(p-dioxanone).

**19.** The monofilament of claim 18 wherein the crystalline structure of the inner core is characterized by larger and more spherolitic crystals as compared to the annular portion.

**20.** The monofilament of claim 18, having an elongation greater than 45%, a Young's modulus of less than $1.7x10^6$kPa (250,000 psi), and a ratio of Tenacity/Modulus greater than 0.30.

**21.** A surgical suture comprising a monofilament of any of claims 16 to 20 having a diameter of from about 7.6 to 610µm (0.3 to 24 mils).

**Patentansprüche**

**1.** Verfahren zum Herstellen eines Monofilaments aus einer thermoplastischen Polymerzusammensetzung, mit den Schritten des Schmelzextrudierens und Abschreckens des Polymers, um ein fortlaufendes Filament zu bilden, des Ziehens des Filaments, um eine molekulare Orientierung zu erhalten, und des Temperaturbehandelns des Filaments während oder nach dem Ziehen des Filaments bei einer Temperatur, die über der Schmelztemperatur der Polymerzusammensetzung liegt, wobei das Filament nach dem Ziehen und der Temperaturbehandlung entspannt oder ausgeheizt wird, und wobei die Temperaturbehandlung für eine Zeitspanne und bei einer Temperatur erfolgt, die dazu führt, daß ein Filament mit einer Außenseite, einem zentralen Kern und einem ringförmigen Abschnitt zwischen dem zentralen Kern und der Außenseite erhalten wird, wobei der zentrale Kern eine Kristallstruktur hat, die sich dadurch von der des ringförmigen Abschnitts unterscheidet, daß eine höher geordnete Kristallstruktur vorliegt als im ringförmigen Abschnitt oder größere Kristalle vorliegen als im ringförmigen Abschnitt, und wobei der Unterschied zwischen dem zentralen Kern und dem ringförmigen Abschnitt durch optische Mikroskopie bei einer Vergrößerung von 430 unter polarisiertem Licht sofort sichtbar ist.

**2.** Verfahren nach Anspruch 1, wobei die Temperaturbehandlung während des ersten Ziehens des extrudierten und abgeschreckten Filaments erfolgt.

**3.** Verfahren nach Anspruch 1, wobei die Temperaturbehandlung nach dem ersten Ziehen des extrudierten und abgeschreckten Filaments erfolgt und das Filament während der Temperaturbehandlung unter Spannung gehalten wird.

**4.** Verfahren nach Anspruch 1, wobei das Filament während eines ersten Ziehens des extrudierten und abgeschreckten Filaments temperaturbehandelt wird und während eines zweiten Ziehens des gezogenen Filaments ein zweites Mal temperaturbehandelt wird.

**5.** Verfahren nach Anspruch 1, wobei das erste Ziehen mit mehr als 200 Prozent und das zweite Ziehen mit weniger als 100 Prozent erfolgt.

**6.** Verfahren nach Anspruch 1, wobei der ringförmige Abschnitt eine radiale Dicke von etwa dem 0,1- bis 0,5-fachen des Radiusses des Filaments hat.

**7.** Verfahren nach Anspruch 1, wobei die Temperatur der Temperaturbehandlung etwa 5 bis 100°C über der Schmelztemperatur des Polymers liegt.

**8.** Verfahren nach Anspruch 7, wobei die Zeitspanne der Temperaturbehandlung von 0,1 bis 5 Sekunden reicht.

**9.** Verfahren nach Anspruch 1, wobei das thermoplastische Polymer aus der Gruppe ausgewählt wird, die Polyethylen, Polypropylen, Polyvinylidenfluorid, Poly-(Glykolid, Cotrimethylcarbonat), Homopolymere und Copolymere von Laktid und Glykolid und Copolymere von p-Dioxanon mit Laktid, Glykolid und ε-Caprolacton umfaßt.

**10.** Verfahren nach Anspruch 1, wobei das Polymer Poly-(p-Dioxanon) ist.

**11.** Verfahren nach Anspruch 10, wobei das extrudierte und abgeschreckte Monofilament um das 4- bis 6-fache gezogen wird, während es einer Temperatur von etwa 105°C bis 175°C für eine Zeitspanne von etwa 0,1 bis 5 Sekunden ausgesetzt ist.

12. Verfahren nach Anspruch 11, wobei das Filament um das 5-fache gezogen wird, während es einer Temperatur von etwa 120 bis 140°C ausgesetzt ist.

13. Verfahren nach Anspruch 11, wobei das gezogene und temperaturbehandelte Filament um etwa 5 bis 30% nachgezogen wird, während es ein zweites Mal einer Temperatur von etwa 105 bis 175°C für eine Zeitspanne von etwa 0,1 bis 5 Sekunden ausgesetzt wird.

14. Verfahren nach Anspruch 13, wobei das nachgezogene und temperaturbehandelte Filament durch Schrumpfenlassen um 10 bis 25% der Nachziehlänge bei einer Temperatur unter dem Schmelzpunkt entspannt wird.

15. Verfahren nach Anspruch 14, wobei das Filament nach dem Entspannen bei einer Temperatur von etwa 70 bis 90°C ausgeheizt wird, wobei es gegen ein weiteres Schrumpfen eingespannt ist.

16. Gezogenes und orientiertes Monofilament aus einem thermoplastischen Polymer mit einem Aufbau, der durch eine Außenseite, einem zentralen Kern und einem ringförmigen Abschnitt zwischen dem zentralen Kern und der Außenseite bestimmt ist, wobei der ringförmige Abschnitt eine radiale Abmessung von etwa dem 0,1- bis 0,5-fachen des Radiusses des Monofilaments aufweist und der zentrale Kern eine Kristallstruktur besitzt, die sich von der des ringförmigen Abschnitts dadurch unterscheidet, daß eine höher geordnete Kristallstruktur vorliegt als im ringförmigen Abschnitt oder größere Kristalle vorliegen als im ringförmigen Abschnitt, und wobei der Unterschied zwischen dem zentralen Kern und dem ringförmigen Abschnitt durch optische Mikroskopie bei einer Vergrößerung von 430 unter polarisiertem Licht sofort sicht-bar ist.

17. Monofilament nach Anspruch 16, wobei das Polymer aus der Gruppe ausgewählt wird, die Polyethylen, Polypropylen, Polyvinylidenfluorid, Poly-(Glykolid, Cotrimethylcarbonat), Homopolymere und Copolymere von Laktid und Glykolid und Copolymere von p-Dioxanon mit Laktid, Glykolid und ε-Caprolacton umfaßt.

18. Monofilament nach Anspruch 16, wobei das Polymer Poly-(p-Dioxanon) ist.

19. Monofilament nach Anspruch 18, wobei die Kristallstruktur des inneren Kerns im Vergleich zum ringförmigen Abschnitt durch größere und sphärolithischere Kristalle charakterisiert ist.

20. Monofilament nach Anspruch 18, mit einer Dehnung von mehr als 45%, einem Elastizitätsmodul von weniger als 1,7 x $10^6$ kPa (250 000 psi) und einem Verhältnis der Festigkeit zum Elastizitätsmodul von mehr als 0,30.

21. Chirurgisches Nahtmaterial mit einem Monofilament nach einem der Ansprüche 16 bis 20 mit einem Durchmesser von etwa 7,6 bis 610 µm (0,3 bis 24 mil).

## Revendications

1. Procédé pour produire une structure de monofilament d'une composition polymère thermoplastique comprenant les étapes consistant à extruder à l'état fondu et à tremper le polymère pour former un filament continu, à étirer le filament pour réaliser une orientation moléculaire, et à exposer le filament à une température au-dessus de la température de fusion de la composition polymère pendant ou après l'étirage dudit filament, dans lequel ledit filament est détendu ou recuit après ledit étirage et exposition, et en ce que ladite exposition a lieu pendant une certaine durée et à une température efficace pour obtenir un filament ayant une surface extérieure, un coeur central et une portion annulaire entre ledit coeur central et ladite surface extérieure, ledit coeur central ayant une structure cristalline qui est différenciée de celle de ladite portion annulaire en ayant une structure cristalline à ordre plus élevé que ladite portion annulaire, ou des cristaux plus grands que ladite portion annulaire, et ladite différenciation entre ledit coeur central et ladite portion annulaire étant facilement visible par microscopie optique à un grossissement de 430 x sous lumière polarisée.

2. Procédé selon la revendication 1, dans lequel ladite exposition a lieu pendant le premier étirage du filament extrudé et trempé.

3. Procédé selon la revendication 1, dans lequel ladite exposition a lieu après le premier étirage dudit filament extrudé et trempé, et ledit filament est maintenu sous tension mécanique pendant ladite exposition.

**4.** Procédé selon la revendication 1, dans lequel ledit filament est exposé une première fois pendant un premier étirage dudit filament extrudé et trempé, et est exposé une deuxième fois pendant un deuxième étirage dudit filament étiré.

**5.** Procédé selon la revendication 1, dans lequel ledit premier étirage dépasse 200 pourcent et ledit deuxième étirage est inférieur à 100 pourcent.

**6.** Procédé selon la revendication 1, dans lequel la portion annulaire possède une épaisseur radiale d'environ 0,1 à 0,5 x le rayon du filament.

**7.** Procédé selon la revendication 1, dans lequel ladite température d'exposition est d'environ 5 à 100°C supérieure à la température de fusion dudit polymère.

**8.** Procédé selon la revendication 7, dans lequel la durée de ladite exposition est d'environ 0,1 à 5 secondes.

**9.** Procédé selon la revendication 1, dans lequel ledit polymère thermoplastique est choisi dans le groupe formé par le polyéthylène, le polypropylène, le poly(fluorure de vinylidène), le poly(glycolide, cocarbonate de triméthyle), les homopolymères et les copolymères de lactide et de glycolide et les copolymères de p-dioxanone avec un lactide, un glycolide et la ε-caprolactone.

**10.** Procédé selon la revendication 1, dans lequel ledit polymère est la poly(p-dioxanone).

**11.** Procédé selon la revendication 10, dans lequel ledit monofilament extrudé et trempé est étiré 4 à 6 x tout en étant exposé à une température comprise entre environ 105°C et 175°C pendant une durée d'environ 0,1 à 5 secondes.

**12.** Procédé selon la revendication 11, dans lequel ledit filament est étiré 5 x tout en étant exposé à une température d'environ 120 à 140°C.

**13.** Procédé selon la revendication 11, dans lequel ledit filament étiré et exposé est étiré de nouveau d'environ 5 à 30% tout en étant exposé une deuxième fois à une température d'environ 105 à 175°C pendant une durée d'environ 0,1 à 5 secondes.

**14.** Procédé selon la revendication 13, dans lequel ledit filament ré-étiré et exposé se détend, sa longueur se rétrécissant de 10 à 25% à une température au-dessous de son point de fusion.

**15.** Procédé selon la revendication 14, dans lequel ledit filament est recuit après détente à une température comprise entre environ 70 et 90°C tout en étant empêché de subir un rétrécissement supplémentaire.

**16.** Structure de monofilament étiré et orienté comprenant un polymère thermoplastique et ayant une structure définie par une surface extérieure, un coeur central, et une portion annulaire entre ledit coeur central et ladite surface extérieure, ladite portion annulaire ayant une dimension radiale d'environ 0,1 à 0,5 x le rayon du monofilament, ledit noyau central ayant une structure cristalline qui est différenciée de celle de ladite portion annulaire en ayant une structure cristalline à ordre plus élevé que ladite portion annulaire, ou des cristaux plus grands que la portion annulaire, et ladite différenciation entre ledit coeur central et ladite portion annulaire étant facilement visible par microscopie optique à un grossissement de 430 x sous lumière polarisée.

**17.** Monofilament selon la revendication 16, dans lequel ledit polymère est choisi dans le groupe formé par le polyéthylène, le polypropylène, le poly(fluorure de vinylidène), le poly(glycolide, cocarbonate de triméthyle), les homopolymères et les copolymères de lactide et de glycolide et les copolymères de p-dioxanone avec un lactide, un glycolide et la ε-caprolactone.

**18.** Monofilament selon la revendication 16, dans lequel ledit polymère est la poly(p-dioxanone).

**19.** Monofilament selon la revendication 18, dans lequel la structure cristalline du coeur interne est caractérisée par des cristaux plus grands et plus sphérolithiques par rapport à la portion annulaire.

**20.** Monofilament selon la revendication 18, ayant un allongement supérieur à 45%, un module de Young inférieur à $1,7 \times 10^6$ kPa (250 000 psi), et un rapport de ténacité/module supérieur à 0,30.

21. Suture chirurgicale comprenant un monofilament selon l'une quelconque des revendications 16 à 20, ayant un diamètre compris entre environ 7,6 et 610 µm (0,3 et 24 mils).

FIG-1
FIG-2
SPOOL
F
TO RACK ANNEALING
10
11
12
13
14
15
16
17
18
19
20
21
22
23
24
25
26
27

FIG-3

A

FIG-4

FIG-5

B

FIG-6